(19) Europäisches Patentamt
European Patent Office
Office européen des brevets

(11) Veröffentlichungsnummer : **0 146 859**
**B1**

(12) # EUROPÄISCHE PATENTSCHRIFT

(45) Veröffentlichungstag der Patentschrift : **04.03.87**

(21) Anmeldenummer : **84115033.7**

(22) Anmeldetag : **10.12.84**

(51) Int. Cl.⁴ : **C 07 C 51/12**, C 07 C 57/03, C 07 C 57/26, C 07 C 67/36, C 07 C 69/52, C 07 D309/06, C 07 D307/32

(54) **Verfahren zur Herstellung von 4-substituierten But-3-en-1-carbonsäuren und von deren Estern.**

(30) Priorität : **15.12.83 DE 3345375**

(43) Veröffentlichungstag der Anmeldung : **03.07.85 Patentblatt 85/27**

(45) Bekanntmachung des Hinweises auf die Patenterteilung : **04.03.87 Patentblatt 87/10**

(84) Benannte Vertragsstaaten : **BE CH DE FR GB IT LI NL**

(56) Entgegenhaltungen :
**US-A- 4 189 608**
**ANGEWANDTE CHEMIE, Band 80, Nr. 9, 1968, Seiten 352-359, Weinheim, DE; K. BITTLER et al.: "Carbonylierung von Olefinen bei milden Temperaturbedingungen in Gegenwart von Palladium-Komplexen"**
**ZEITSCHRIFT FÜR NATURFORSCHUNG, 18B, 1963, Seiten 772-773, Tübingen, DE; G.W. PARSHALL: "Zur Carbonylierung von Allylakohol"**

(73) Patentinhaber : **BASF Aktiengesellschaft**
**Carl-Bosch-Strasse 38**
**D-6700 Ludwigshafen (DE)**

(72) Erfinder : **Himmele, Walter, Dr. Chem.**
**Eichenweg 14**
**D-6909 Walldorf (DE)**
Erfinder : **Hoffmann, Werner, Dr. Chem.**
**Ringstrasse 11 c**
**D-6701 Neuhofen (DE)**
Erfinder : **Janitschke, Lothar, Dr. Chem.**
**Wormser Gasse 9 a**
**D-6711 Kleinniedesheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 146 859 B1

**Beschreibung**

Die vorliegende Erfindung betrifft ein neues Verfahren zur Herstellung von 4-substituierten But-3-en-1-carbonsäuren bzw. von deren Estern der allgemeinen Formel I

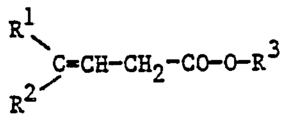

(I)

in welcher $R^1$ einen organischen Rest und $R^2$ Wasserstoff oder einen organischen Rest bedeuten, wobei $R^1$ und $R^2$ auch unter Ausbildung eines 5- bis 20-gliedrigen Ringes miteinander verbunden sein können und wobei diese Reste sich unter den nachstehenden Reaktionsbedingungen indifferent verhalten sollen, und in welcher $R^3$ für Wasserstoff oder eine niedere Alkigruppe steht.

Aus der Arbeit von Tsuji et al in J. Am. Chem. Soc. *86* (1964), Seiten 4 350-53, ist es bekannt, den Ethylester der Pent-3-en-1-carbonsäure (I, $R^1$ = Me, $R^2$ = H, $R^3$ = Et) durch Carbonylierung von But-1-en-3-ol mit Kohlenmonoxid und Ethanol in Gegenwart von Palladiumchlorid als Carbonylierungskatalysator herzustellen. Die hierbei erzielte Ausbeute von lediglich 39 % ist für technische Zwecke jedoch unbefriedigend.

Weiterhin ist es aus der Arbeit von Bittler et al in Angew. Chemie, Band 80 (1968), Seiten 352-359, bekannt, Allylalkohol und Methanol mittels eines Palladiumchlorid/Triphenylphosphinkomplexes in 65 %iger Ausbeute zum Methylester der But-3-en-carbonsäure zu carbonylieren. Nach Aussage der Autoren (s.S. 355 loc.cit.) findet die Carbonylierung des Allylalkohols unter Erhaltung der Doppelbindung statt, so daß man zur Herstellung der 4-substituierten But-3-en-carbonsäureester von den schwer zugänglichen Allylalkoholen

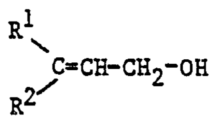

ausgehen müßte.

Der Erfindung lag daher die Aufgabe zugrunde, die teils unmittelbar als Duftstoffe verwendbaren und allgemein für organische Synthesen wichtigen Verbindungen I besser zugänglich zu machen.

Demgemäß wurde im Hinblick auf Bittler et al unerwarteterweise ein Verfahren zur Herstellung der eingangs definierten Verbindungen (I) durch Carbonylierung von Derivaten des Allylalkohols mit Hilfe eines Palladium-halogenids gefunden, welches dadurch gekennzeichnet ist, daß man einen Allylalkohol II

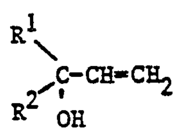

(II)

bei 50-150 °C und unter 200-700 bar mit Kohlenmonoxid

a) zur Herstellung von überwiegenden Mengen an Estern I mit einem Alkohol III

$$R^{3'}—OH \qquad (III)$$

in welchem $R^{3'}$ für eine niedere Alkylgruppe steht, oder

b) zur Herstellung der Säuren I ohne einen weiteren Reaktionspartner

in Gegenwart wirksamer Mengen eines Komplexes aus einem Palladiumhalogenid und einem tertiären Phosphin (IV) carbonyliert.

Die Ausgangsverbindungen II sind auf einfache Weise aus den entsprechenden Carbonylverbindungen $R^1—CO—R^2$ und Vinylmagnesiumhalogeniden erhältlich. Weiterhin kann man II aus den Carbonylverbindungen und Acetyliden wie Natriumacetylid und anschließende partielle Hydrierung der Ethinylgruppe zur Vinylgruppe herstellen.

Nach den bisherigen sehr umfangreichen Untersuchungen kommt es bei dem erfindungsgemäßen Verfahren auf die Natur der Verbindungen II, also auf die Art der organischen Reste $R^1$ und $R^2$ grundsätzlich nicht an, sofern diese Reste keine Substituenten oder Gruppierungen enthalten, die unter den Carbonylierungsbedingungen nicht indifferent sind. Solche nicht-indifferenten Reste sind beispielsweise Alkenylgruppen mit endständiger Doppelbindung ; ist die Doppelbindung hingegen innenständig, wird sie unter den Reaktionsbedingungen in aller Regel praktisch nicht angegriffen.

Als Beispiele für geeignete Reste $R^1$ und $R^2$ seien genannt :

aliphatische Reste mit 1-30 C Atomen
cycloaliphatische Reste mit 3-12 Ringgliedern
aromatische Reste mit 1-3 iso- oder heterocyclischen aromatischen Ringen
gemischte Reste mit aliphatischen und/oder cycloaliphatischen und/oder aromatischen Gruppierungen in beliebiger Reihenfolge und
gemeinsam cyclische Reste mit 3-20 Ringgliedern, die ihrerseits mit den vorgenannten Resten substituiert sein können.

All diese Reste können ferner Substituenten wie Halogen, z. B. Fluor and Chlor, die Hydroxylgruppe (die im allgemeinen bei der erfindungsgemäßen Carbonylierung praktisch nicht angegriffen wird), substituierte Aminogruppen, die Cyangruppe, die Nitrogruppe, Alkoxy-, Acyl-, Acyloxy- und Carbalkoxygruppen, die Thiolgruppe, Mercaptangruppen, die Carbonamidgruppe und Alkylsulfonylgruppen enthalten. Besondere Bedeutung kommt dejenigen Alkoholen II zu, die in ihrem Molekül ein- oder mehrfach das Isoprengerüst enthalten : die hiervon abgeleiteten Verbindungen I haben häufig ausgeprägten Duftstoffcharakter.

Als Alkohole III kommen vornehmlich niedere Alkohole wie Methanol und Ethanol in Betracht, da mit diesen die Carbonylierung am schnellsten verläuft. Höhere Alkanole mit etwa bis zu 6 C-Atomen können prinzipiell auch verwendet werden, jedoch ist es häufig zweckmäßiger, diese durch Umesterung der Methyl- oder Ethylester I in das Molekül einzuführen.

Im allgemeinen empfiehlt es sich, den Alkohol III im molaren Überschuß über den Alkohol II einzusetzen. Üblicherweise liegt das Molverhältnis von III zu II zwischen 2 : 1 und 6 : 1. Dient III als Lösungsmittel, kann dieses Verhältnis auch höher sein.

Arbeitet man in Abwesenheit weiterer Reaktionspartner, also ohne einen Alkohol III, so erhält man die Säuren I. Überwiegende Mengen an Säure erhält man meistens auch bei Verwendung eines tertiären Alkohols III wie tert.-Butanol.

Die Carbonylierungskatalysatoren sind Komplexverbindungen aus einem Palladiumhalogenid, z. B. dem Bromid und besonders dem Chlorid einerseits und einem tertiären organischen Phosphin (IV) andererseits. Da diese Komplexe unter den Reaktionsbedingungen von selber entstehen, setzt man sie zweckmäßigerweise in Form ihrer Komponenten ein, jedoch ist es selbstverständlich auch möglich, von den vorgebildeten Komplexen, z. B. $Pd(PPh_3)_2Cl_2$ (Ph = Phenyl), auszugehen.

Die tertiären Phosphine IV entsprechen der allgemeinen Formel IV

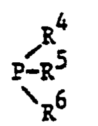

(IV)

in der $R^4$ bis $R^6$ organische Reste bedeuten.

Insbesondere können $R^4$ bis $R^6$ Alkylgruppen mit 4 bis 18 C-Atomen sowie $C_1$- bis $C_4$-Alkylphenylgruppen und vor allem die Phenylgruppe bedeuten. Zwei dieser Reste können auch miteinander unter Ausbildung eines 5- bis 8-gliedrigen Ringes miteinander verbunden sein, und selbstverständlich kommen auch Verbindungen mit 2 oder mehreren tert.-Phosphingruppierungen in Betracht, z. B. das 1,2-Bis-(diphenylphosphino)-ethan.

Auf die Art der Phosphine kommt es prinzipiell nicht an, so daß sich im allgemeinen die Verwendung des billigsten Vertreters dieser Stoffklasse, nämlich des Triphenylphosphins, empfiehlt.

Weiterhin ist es zweckmäßig, die Reaktion in Gegenwart von freiem, also nicht im Komplex gegundenem Phosphin auszuführen. Hierbei empfiehlt sich ein Molverhältnis von Phosphin zu Pd von 3 : 1 bis 10 : 1.

Für die wirksamen Mengen des Katalysators lassen sich keine bestimmten Werte angeben, weil die Reaktion prinzipiell mit beliebig geringen Mengen Pd abläuft, dann aber natürlich recht langsam ist, und weil große Mengen nicht schaden. Die Menge des Pd richtet sich daher nach der Reaktivität des Alkohols II und der angestrebten Reaktionszeit. Im allgemeinen liegt diese Menge zwischen 0,1-5 g Pd pro Mol II.

Entsprechend den allgemeinen Grundsätzen der Verfahrenstechnik gelingt die Reaktion am besten und schnellsten, wenn sie in weitgehend homogener flüssiger Phase ablaufen kann. Meistens stellt sich die Homogenität wegen der Anwesenheit des Alkohols III unter den Reaktionsbedingungen von selbst ein, so daß kein zusätzliches Lösungsmittel verwendet zu werden braucht. Ist der Alkohol II jedoch schwerlöslich, ist die Verwendung eines Lösungsmittels wie Toluol, Diethylether oder Tetrahydrofuran ratsam.

Der Druck — er entspricht größtenteils dem CO-Partialdruck — liegt im allgemeinen zwischen 100 und 650, besonders zwischen 300 und 600 bar, und für die Temperatur empfiehlt sich ein Bereich von 50-150, vorzugsweise 90-110 °C. Unter diesen Bedingungen sowie mit den empfohlenen Katalysatormengen betragen die Reaktionszeiten etwa 1-24 Stunden.

Methodisch gleicht das erfindungsgemäße Verfahren, welches kontinuierlich oder diskontinuierlich ausgeführt werden kann, der üblichen Carbonylierungstechnik, so daß nähere Ausführungen hierzu entbehrlich sind. Das gleiche gilt für die Aufarbeitung der Reaktionsgemische. Wegen der Oxidationsempfindlichkeit der Phosphine empfiehlt es sich, unter Ausschluß von Luftsauerstoff zu arbeiten.

Als Verfahrensprodukte erhält man bei Mitverwendung des Alkohols III hauptsächlich die Ester I sowie auch die freien Säuren I : tertiäre Alkohole III liefern meist größere Mengen der Säure. Der Anteil der Nebenprodukte ist durchweg gering, obwohl zu erwarten war, daß die Alkohole II unter den Reaktionsbedingungen dehydratisiert werden. Die Verbindungen I können sich in der cis- und der trans-Form ausbilden ; normalerweise erhält man das trans-Isomere in überwiegenden Anteilen.

## Beispiele

Verschiedene Alkohole II wurden jeweils unter Ausschluß von Luftsauerstoff bei 100 °C in Gegenwart von Palladiumchlorid und Triphenylphospin ($PPh_3$) mit verschiedenen Alkoholen III zu den Säuren bzw. Estern I umgesetzt.

Einzelheiten zu den sonstigen Reaktionsbedingungen sowie zu den Ergebnissen der Versuche sind der nachstehenden Tabelle zu entnehmen. Etwa 98 % des angegebenen Druckes entfallen auf den CO-Partialdruck. Die Reaktion wurde jeweils dann abgebrochen, wenn kaum noch CO verbraucht wurde.

Die Produkte wurden mit Hilfe der GC—, H—NMR— und $^{13}C$—NHR—, MS— und IR-Analyse identifiziert. Die Ausbeuten wurden gravimetrisch bestimmt ; soweit die Trennung in Säure und Ester Schwierigkeiten bereitete, wurde lediglich die Summe der Ausbeuten ermittelt. Siedepunkte und Schmelzpunkte wurden in den Fällen angegeben, in denen sie sich unmittelbar aus den Versuchen ergaben.

(Siehe Tabelle Seite 5 ff.)

| Bsp. | Alkohol II [g] | Alkohol III [g] | $PdCl_2$ [g] $PPh_3$ [g] | Druck [bar] | Dauer [h] | Ausbeuten Gesamtausbeute | Säure/Ester I, trans/cis-Verh. q, Charakteristika |
|---|---|---|---|---|---|---|---|
| 1 | Pent-1-en-3-ol<br>100 | MeOH<br>50 | 1,0<br>3,0 | 600 | 17 | 14 %<br>78 %<br>92 % | Hex-3-en-säure;<br>- methylester, Sdp. 100-101°C/231 mbar<br>q = 85:15 |
| 2 | Hex-1-en-3-ol<br>100 | EtOH<br>50 | 1,0<br>3,0 | 600 | 8 | 15 %<br>54 %<br>69 % | Hept-3-en-säure;<br>- ethylester, Sdp. 89°C/45 mbar<br>q = 85:15<br>Geruch Säure: grünlich, fruchtig, etw.stechend<br>- Ester: süßlich, blumig, etwas tonkaartig |
| 3 | Hept-1-en-3-ol<br>920 | EtOH<br>500 | 5,0<br>1,5 | 300 | 15 | 18 %<br>69 %<br>87 % | Oct-3-en-säure;<br>- ethylester, Sdp. 112-114°C/54 mbar<br>q = 80:20<br>Geruch Säure und Ester: grünlich, leicht fettig |
| 4 | Oct-1-en-3-ol<br>730 | n-Propanol<br>500 | 5,0<br>15,0 | 300 | 8 | 15 %<br>64 %<br>79 % | Non-3-en-säure;<br>- n-propylester, Sdp. 70-72°C/4 mbar<br>q = 82:18<br>Geruch Ester: grün, schwach krautig, pilzartig |
| 5 | 3-Methylbut-1-en-3-ol<br>1000 | MeOH<br>500 | 2,5<br>7,5 | 300 | 8 | 9 %<br>47 %<br>56 % | 4-Methylpent-3-en-säure; Sdp. 122°C/48 mbar<br>- methylester, Sdp. 110°C/150 mbar |
| 6 | 3,7-Dimethyloct-1-en-3-ol<br>1000 | 1-Propanol<br>500 | 2,5<br>7,5 | 300 | 8 | 23 %<br>57 %<br>80 % | 4,8-Dimethylnon-3-en-säure; Sdp.165°C/45 mbar<br>- isopropylester, Sdp. 145°C/37 mbar<br>q = 87:13 |

| Bsp. | Alkohol II [g] | Alkohol III [g] | $PdCl_2$ [g] $PPh_3$ [g] | Druck [bar] | Dauer [h] | Ausbeuten Säure/Ester I Gesamtausbeute, trans/cis-Verh. q Charakteristika | |
|---|---|---|---|---|---|---|---|
| 7 | 3,7-Dimethyloct-1-en-3-ol<br>1000 | n-Propanol<br>500 | 5<br>15 | 300 | 8 | 13 %<br>68 %<br>81 % | 4,8-Dimethylnon-3-en-säure;<br>- n-propylester, Sdp. 147°C/34 mbar<br>cis-Ester: süßlich, balsamisch, fruchtig<br>trans-Ester: blumig, etwas fettig |
| 8 | 3,7-Dimethyloct-1-en-3-ol<br>1000 | n-Butanol<br>5000 | 5<br>15 | 300 | 15 | 17 %<br>63 %<br>80 % | 4,8-Dimethylnon-3-en-säure;<br>- n-butylester, Sdp. 160°C/37 mbar<br>q = 66:34 |
| 9 | 3,7-Dimethylocta-1,6-dien-3-ol<br>1000 | EtOH<br>500 | 5<br>15 | 250 | 14 | 22 %<br>61 %<br>83 % | 4,8-Dimethylnona-3,7-dien-säure;<br>- ethylester, Sdp. 82-84°C/4 mbar<br>q = 68:32<br>cis-Ester: fruchtig, grünlich<br>trans-Ester: angenehm fruchtig, moschusartig |
| 10 | 3,7-Dimethylocta-1,6-dien-3-ol<br>1000 | EtOH<br>500 | 2,5<br>7,5 | 300 | 10 | 34 %<br>56 %<br>90 % | 4,8-Dimethylnona-3,7-dien-säure;<br>- ethylester, Sdp. 82°C/4 mbar |
| 11 | 3,7,11-Trimethyldodec-1-en-3-ol<br>410 | EtOH<br>205 | 2,5<br>7,5 | 300 | 6 | 3 %<br>90 %<br>93 % | 4,8,12-Trimethyltridec-3-en-säure;<br>- ethylester Sdp. 125-130°C/4 mbar<br>q = 80:20 |
| 12 | 3,7,11-Trimethyl-dodeca-1,6-dien-3-ol (Dihydronerolidol)<br>1125 | MeOH<br>500 | 2,5<br>7,5 | 300 | 6 | 11 %<br>63 %<br>74 % | 4,8,12-Trimethyltrideca-3,7-dien-säure;<br>- methylester, Sdp. 125-129°C/4 mbar |

| Bsp. | Alkohol II [g] | Alkohol III [g] | $PdCl_2$ [g] $PPh_3$ [g] | Druck [bar] | Dauer [h] | Ausbeuten Gesamtausbeute | Säure/Ester I, trans/cis-Verh. q, Charakteristika |
|---|---|---|---|---|---|---|---|
| 13 | 3,7,11-Trimethyl-dodeca-1,6-dien-3-ol (Dihydronerolidol) 1000 | EtOH 500 | 5 / 15 | 300 | 8 | 6 % / 81 % / 87 % | 4,8,12-Trimethyltrideca-3,7-dien-säure; - ethylester |
| 14 | 3,7,11-Trimethyl-dodeca-1,6,10-trien-3-ol (Nerolidol) 1000 | MeOH 500 | 2,5 / 7,5 | 300 | 10 | 7 % / 79 % / 86 % | 4,8,12-Trimethyltrideca-3,7,11-trien-säure; - methylester, Sdp. 129-130°C/4 mbar |
| 15 | 3,7,11-Trimethyl-dodeca-1,6,10-trien-3-ol (Nerolidol) 1000 | EtOH 500 | 2,5 / 7,5 | 300 | 14 | 7 % / 79 % / 86 % | 4,8,12-Trimethyltrideca-3,7,11-trien-säure; - ethylester, Sdp. 126-130°C/4 mbar |
| 16 | 3,7,11-Trimethyl-dodeca-1,6,10-trien-3-ol (Nerolidol) 1000 | EtOH 500 | 1,25 / 3,75 | 300 | 17 | 9 % / 69 % / 77 % | 4,8,12-Trimethyltrideca-3,7,11-trien-säure; - ethylester, Sdp. 127-152°C/4 mbar |
| 17 | 3,7,11,15-Tetramethylhexadec-1-en-3-ol (Isophytol) 1000 | EtOH 500 | 2,5 / 7,5 | 300 | 19 | 4 % / 90 % / 94 % | 4,8,12,16-Tetramethylheptadec-3-en-säure; - ethylester, Sdp. 162-167°C/4 mbar; q = 3:1 |
| 18 | 5-(2,6,6-Trimethylcyclohex-1-en-1-yl)-3-methylpent-1-en-3-ol 600 | EtOH 300 | 3,5 / 10,0 | 300 | 4 | 8 % / 76 % / 84 % | 6-(2,6,6-Trimethylcyclohex-1-yl)-4-methylhex-3-en-säure; - ethylester, Sdp. 148-157°C/5 mbar; q = 82:18; cis-Ester: süßlich-fruchtig (Apfel, Erdbeer) |

| Bsp. | Alkohol II [g] | Alkohol III [g] | $PdCl_2$ [g] $PPh_3$ [g] | Druck [bar] | Dauer [h] | Ausbeuten | Säure/Ester I Gesamtausbeute, trans/cis-Verh. q Charakteristika |
|---|---|---|---|---|---|---|---|
| 19 | 5-(2,6,6-Trimethylcyclohex-1-en-1-yl)-3-methylpenta-1,4-dien-3-ol (Vinylionol) 100 | EtOH 50 | 1,0<br>3,0 | 600 | 10 | *)<br><br><br>25 % | 6-(2,6,6-Trimethylcyclohex-1-en-yl)-4-methylhexa-3,5-dien-säure;<br>- ethylester, Sdp. 159-162°C<br>Mischung: fruchtig, damasconartig, Aprikose |
| 20 | 3-Ethylpent-1-en-3-ol 120 | EtOH 60 | 1,0<br>3,0 | 600 | 22 | 11 %<br>54 %<br>65 % | 4-Ethylpent-3-en-säure;<br>- ethylester, Sdp. 106-107°C/52 mbar<br>Ester: stechend, grünlich, fruchtig |
| 21 | 4-Vinylheptan-4-ol 100 | EtOH 50 | 1,0<br>3,0 | 600 | 17 | 6 %<br>56 %<br>62 % | 4-n-Propylhept-3-en-säure;<br>- ethylester, Sdp. 53-55°C/4 mbar<br>Ester: Fruchtig, grün, gummiartig |
| 22 | 1-Vinylcyclodedecan-1-ol 100 | EtOH 50 | 1,0<br>3,0 | 600 | 23 | 8 %<br>61 %<br>69 % | 3-(Cyclododecyliden)-propion-säure;<br>- ethylester, Sdp. 144-146°C/4 mbar<br>Ester: fettig, säuerlich, schwach süßlich, balsamisch |
| 23 | 2-Methyl-1-vinyl-cyclohexan-1-ol 93,5 | EtOH 46,7 | 1,0<br>3,0 | 600 | 17 | 5 %<br>51 %<br>56 % | 3-(2-Methylcyclohexyliden)-propion-säure;<br>- ethylester, Sdp. 116-122°C/28 mbar |
| 24 | 3-Methyl-1-vinyl-cyclohexan-1-ol 92 | EtOH 46 | 1,0<br>3,0 | 600 | 34 | 5 %<br>73 %<br>78 % | 3-(3-Methylcyclohexyliden)-propion-säure;<br>- ethylester, Sdp. 105-110°C/16 mbar<br>Ester: fruchtig, süßlich, fettalkoholartig |

*) Einzelausbeuten nicht bestimmt.

| Bsp. | Alkohol II [g] | Alkohol III [g] | $PdCl_2$ [g]<br>$PPh_3$ [g] | Druck [bar] | Dauer [h] | Ausbeuten Säure/Ester I<br>Gesamtausbeute | trans/cis-Verh. q<br>Charakteristika |
|---|---|---|---|---|---|---|---|
| 25 | 1-Vinyl-cyclohexan-1-ol<br>876 | EtOH<br>500 | 2,5<br>7,5 | 300 | 17 | 12 %<br>58 %<br>70 % | 3-(Cyclohexyliden)-propion-säure;<br>- ethylester, Sdp. 130°C/30 mbar<br>Ester: fruchtig, erdbeerartig, säuerlich |
| 26 | 3,3-Dimethyl-1-vinylcyclo-hexan-1-ol<br>90 | EtOH<br>50 | 1,0<br>3,0 | 600 | 7 | 3 %<br>76 %<br>79 % | 3-(3,3-Dimethylcyclohexyliden)-propionsäure;<br>- ethylester, Sdp. 138-140°C/40 mbar<br>q = 50:50<br>Ester: holzig, fruchtig, grünlich |
| 27 | Gemisch aus 2,2,4- und 2,4,4-Trimethyl-1-vinyl-cyclopentan-1-ol<br>100 | EtOH<br>50 | 1,0<br>3,0 | 600 | 16 | 4 %<br><br>58 %<br>62 % | 3-(2,2,4/2,4,4-Trimethylcyclo-pentyliden)-propionsäure;<br>- ethylester, Sdp. 62-63°C/4 mbar<br>Ester: grün, erdig, pilzig, holzig |
| 28 | 1-Vinyl-1-hydroxy-1,2,3,4-tetrahydronaphthalin<br>100 | EtOH<br>100 | 1,0<br>3,0 | 600 | 13 | 5 %<br>60 %<br>65 % | 2,3-Benzocyclohexylid-1-en-propion-säure;<br>- ethylester, Sdp. 110°C/4 mbar<br>q = 70:30 |
| 29 | 9-Vinyl-9-hydroxyfluoren<br>85 | EtOH<br>50 | 1,0<br>3,0 | 600 | 10 | <1 %<br>83 %<br>83 % | 3-(Fluorenylid-9-en)-propionsäure;<br>- ethylester, Sdp. 60-65°C |
| 30 | 3,3-Diphenylprop-1-en-3-ol<br>73 | EtOH<br>50 | 1,0<br>3,0 | 600 | 10 | 8 %<br>72 %<br>80 % | 4,4-Diphenylbut-3-en-säure;<br>- ethylester, Sdp. 168°C/4 mbar |

| Bsp. | Alkohol II [g] | Alkohol III [g] | $PdCl_2$ [g] / $PPh_3$ [g] | Druck [bar] | Dauer [h] | Ausbeuten Gesamtausbeute | Säure/Ester I, trans/cis-Verh. q, Charakteristika |
|---|---|---|---|---|---|---|---|
| 31 | 3-Phenylbut-1-en-3-ol<br>70 | EtOH<br>50 | 1,0<br>3,0 | 600 | 8 | 5 %<br>40 %<br>30 %<br>75 % | 4-Phenyl-but-3-en-säure;<br>- ethylester, Sdp. 159°C/40 mbar<br>2-Methyl-2-phenylbutyrolacton,<br>Sdp. 165-167°C/40 mbar |
| 32 | 3-(3,3-Dimethylcyclo-hex-1-yl)-but-1-en<br>100 | EtOH<br>50 | 1,0<br>3,0 | 600 | 5 | 8 %<br>65 %<br>73 % | 4-(3,3-Dimethylcyclohex-1-yl)-pent-3-en-säure;<br>-ethylester, Sdp. 80-82°C/4 mbar<br>q = 1:1 |
| 33 | 3-Cyclopropylbut-1-en-3-ol<br>100 | EtOH<br>50 | 1,0<br>3,0 | 600 | 15 | 4 %<br>38 %<br>42 % | 4-Cyclopropylbut-3-en-säure;<br>- ethylester, Sdp. 113-115°C/44 mbar |
| 34 | 3-Methoxybut-1-en-3-ol<br>80 | EtOH<br>50 | 1,0<br>3,0 | 530 | 12 | 4 %<br>38 %<br>42 % | 4-Methoxypent-3-en-säure;<br>- ethylester, Sdp. 117-125°C/74 mbar<br>q = 80:20 |
| 35 | 3-Methyl-4-phenyl-but-1-en-3-ol<br>101 | EtOH<br>50 | 1<br>3 | 680 | 20 | 12 %<br>68 %<br>80 % | 4-Methyl-5-phenyl-but-3-en-säure;<br>- ethylester, Sdp. 98-99°C/4 mbar<br>q = 65:35<br>Ester: grün, Pelargoniumblätter |
| 36 | 1-Vinyl-cycloheptan-1-ol<br>105 | EtOH<br>45 | 1<br>3 | 600 | 13 | 11 %<br>58 %<br>69 % | 3-Cycloheptyliden-propionsäure;<br>- ethylester, Sdp. 132°C/24 mbar |

| Bsp. | Alkohol II [g] | Alkohol III [g] | $PdCl_2$ [g] $PPh_3$ [g] | Druck [bar] | Dauer [h] | Ausbeuten Gesamtausbeute | Säure/Ester I, trans/cis-Verh. q, Charakteristika |
|---|---|---|---|---|---|---|---|
| 37 | 3-(4-Methoxyphenyl)-but-1-en-3-ol<br>100 | EtOH<br>50 | 1<br>3 | 300 | 16 | 2 %<br>40 %<br>33 %<br>75 % | 4-(4-Methoxyphenyl)-pent-3-en-säure;<br>- ethylester, Sdp. 157-159°C/40 mbar<br>5-(4-Metoxyphenylmethyl)-butyrolacton, Sdp. 165-167°C/40 mbar |
| 38 | 4-tert.-Butoxy-hex-1-en-3-ol<br>58 | EtOH<br>50 | 0,5<br>1,5 | 300 | 11 | 11 %<br>52 %<br>63 % | 5-tert.-Butoxy-hept-3-en-säure;<br>-ethylester, Sdp. 85-86°C/4 mbar |
| 39 | 3-(2-Methoxyphenyl)-but-1-en-3-ol<br>37 | EtOH<br>50 | 0,5<br>1,5 | 300 | 26 | 8 %<br>72 %<br>80 % | 4-(2-Methoxyphenyl)-pent-3-en-säure;<br>- ethylester, Sdp. 122-128°C/4 mbar |
| 40 | 2-Benzyl-1-vinyl-cyclohexan-1-ol<br>37 | EtOH<br>37 | 1<br>3 | 600 | 26 | 4 %<br>79 %<br>83 % | 3-(2-Benzylcyclohexyliden)-propionsäure;<br>- ethylester, Sdp. 163-182°C/4 mbar |
| 41 | 3-(Tetrahydrofyran-3-yl)-prop-1-en-3-ol<br>100 | EtOH<br>50 | 1<br>3 | 650 | 13 | 11 %<br>66 %<br>77 % | 4-(Tetrahydropyran-3-yl)-but-3-en-säure;<br>- ethylester, Sdp. 72-74°C/4 mbar<br>q = 90:10 |
| 42 | Bis-(4-chlorphenyl)-vinylcarbinol<br>100 | EtOH<br>50 | 1<br>3 | 650 | 7 | 8 %<br>63 %<br>71 % | 4,4-Di-(4-chlorphenyl)-but-3-en-säure;<br>- ethylester, Sdp. 208-212°C/4 mbar |
| 43 | Bis-(4-fluorphenyl)-vinylcarbinol<br>115 | EtOH<br>30 | 1<br>3 | 650 | 6 | 5 %<br>68 %<br>73 % | 4,4-Di-(4-fluorphenyl)-but-3-en-säure;<br>- ethylester, Sdp. 148-153°C/4 mbar |

| Bsp. | Alkohol II [g] | Alkohol III [g] | $PdCl_2$ [g] $PPh_3$ [g] | Druck [bar] | Dauer [h] | Ausbeuten Gesamtausbeute | Säure/Ester I trans/cis-Verh. q Charakteristika |
|---|---|---|---|---|---|---|---|
| 44 | 1-Vinyl-2,3-6,7-dibenzo-cyclohept-4-en-1-ol<br>105 | EtOH<br>50 | 1<br>3 | 650 | 8 | 4 %<br>69 %<br>73 % | 3-(2,3-6,7-Dibenzo-cyclohept-4-en-1-yliden)-propionsäure;<br>- ethylester, Sdp. 168-170°C/4 mbar |
| 45 | 1-Vinyl-2,3-6,7-dibenzo-cycloheptan-1-ol<br>100 | EtOH<br>50 | 1<br>3 | 650 | 6 | 3 %<br>69 %<br>72 % | 3-(2,3-6,7-Dibenzocyclohept-1-yliden-propionsäure;<br>-ethylester, Sdp. 182-185°C/4 mbar |
| 46 | 3-(2-Methoxyphenyl)-prop-1-en-3-ol<br>103 | EtOH<br>50 | 1<br>3 | 650 | 12 | 8 %<br>59 %<br>67 % | 4-(2-Methoxyphenyl)-but-3-en-säure;<br>- ethylester, Sdp. 146-153°C/7 mbar |
| 47 | 3,7-Dimethyl-octa-1,6-dien-3-ol<br>1000 | tert.-ButOH<br>500 | 2,5<br>7,5 | 300 | 9 | 66 %<br>20 %<br>86 % | 4,8-Dimethyl-nona-3,7-dien-säure;<br>- tert.-butylester, Sdp. 97-98°C/6 mbar |
| 48 | 3,7-Dimethyl-oct-1-en-3-ol<br>1000 | tert.-ButOH<br>500 | 2,5<br>7,5 | 300 | 13 | 64 %<br>18 %<br>82 % | 4,8-Dimethyl-non-3-en-säure;<br>- tert.-butylester, Sdp. 88°C/4 mbar |
| 49 | 3-Methyl-but-1-en-3-ol<br>1000 | tert.-ButOH<br>500 | 2,5<br>7,5 | 300 | 22 | 71 %<br>13 %<br>84 % | 4-Methyl-but-3-en-säure;<br>- tert.-butylester, Sdp. 87°C/48 mbar |

| Bsp. | Alkohol II [g] | Alkohol III [g] | $PdCl_2$ [g] $PPh_3$ [g] | Druck [bar] | Dauer [h] | Ausbeuten Säure/Ester I Gesamtausbeute, trans/cis-Verh. q | Charakteristika |
|---|---|---|---|---|---|---|---|
| 50 | 3-Methyl-but-1-en-3-ol<br>1000 | tert.-ButOH<br>500 | 2,5<br>7,5**) | 300 | 41 | 58 %<br>15 %<br>73 % | 4-Methyl-but-3-en-säure;<br>- tert.-butylester, Sdp. 87°C/48 mbar |
| 51 | Oct-1-en-3-ol<br>1000 | tert.-ButOH<br>500 | 2,5<br>7,5 | 300 | 32 | 63 %<br>12 %<br>75 % | Oct-3-en-säure;<br>- tert.-butylester, Sdp. 72°C/6 mbar |
| 52 | 3,7,11-Trimethyl-dodeca-1,6,10-trien-3-ol (Nerolidol)<br>1000 | tert.-ButOH<br>500 | 2,5<br>7,5 | 300 | 32 | 74 %<br>3 %<br>77 % | 4,8,12-Trimethyl-trideca-3,7,11-trien-säure;<br>- tert.-butylester<br>Isomerengemisch |
| 53 | 3,7,11-Trimethyl-dodeca-1,6,10-trien-3-ol (Nerolidol)<br>1000 | tert.-ButOH<br>500 | 2,5<br>15**) | 300 | 28 | 82 %<br>2 %<br>84 % | 4,8,12-Trimethyl-trideca-3,7,11-trien-säure;<br>- tert.-butylester<br>Isomerengemisch |
| 54 | 3,7,11,15-Tetramethyl-hexadec-1-en-3-ol (Isophytol)<br>490 | tert.-ButOH<br>500 | 2,5<br>7,5 | 300 | 8 | 72 %<br>7 %<br>79 % | 4,8,12,16-Tetramethyl-heptadec-3-en-säure;<br>- tert.-butylester<br>Isomerengemisch |
| 55 | Oct-1-en-3-ol<br>1210 | –<br>– | 2,5<br>7,5 | 280 | 15 | 84 % | Non-3-en-säure; Sdp. 93-94°C/4 mbar<br>q = 85:15 |

**) n-Hexyldiphenylphosphin.

| Bsp. | Alkohol II [g] | Alkohol III [g] | $PdCl_2$ [g]<br>$PPh_3$ [g] | Druck [bar] | Dauer [h] | Ausbeuten Säure/Ester I Gesamtausbeute, trans/cis-Verh. q Charakteristika | |
|---|---|---|---|---|---|---|---|
| 56 | 3,7-Dimethyl-octa-1,6-dien-3-ol (Linalool)<br>1500 | – | 2,5<br>7,5 | 280 | 16 | 83 % | 4,8-Dimethyl-nona-3,7-dien-säure;<br>Sdp. 120-124°C/4 mbar<br>q = 82:18 |
| 57 | 3-Methyl-but-1-en-3-ol<br>1500 | –<br>– | 2,5<br>7,5 | 240<br>***) | 16 | 83 % | 4-Methyl-but-3-en-säure; Sdp. 70-71°C/4 mbar |
| 58 | 3,7,11-Trimethyl-dodeca-1,6,10-trien-3-ol<br>560 | –<br>– | 2,5<br>7,5 | 240 | 22 | 70 % | Trimethyl-trideca-3,7,11-trien-säure;<br>Sdp. 165-170°C/1 mbar |

***) Temperatur 80 °C.

**Patentanspruch**

Verfahren zur Herstellung von 4-substituierten But-3-en-1-carbonsäuren bzw. von deren Estern der allgemeinen Formel I

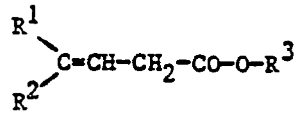

(I)

in welcher $R^1$ einen organischen Rest und $R^2$ Wasserstoff oder einen organischen Rest bedeuten, wobei $R^1$ und $R^2$ auch unter Ausbildung eines 5- bis 20-gliedrigen Ringes miteinander verbunden sein können und wobei diese Reste sich unter den nachstehenden Reaktionsbedingungen indifferent verhalten sollen, und in welcher $R^3$ für Wasserstoff oder eine niedere Alkylgruppe steht, durch Carbonylierung von Derivaten des Allylalkohols mit Hilfe eines Palladiumhalogenids, dadurch gekennzeichnet, daß man einen Alkylalkohol II

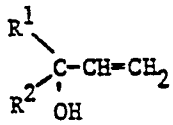

(II)

bei 50-150 °C und unter 200-700 bar mit Kohlenmonoxid

a) zur Herstellung von überwiegenden Mengen an Estern I mit einem Alkohol III

$R^{3'}$—OH (III)

in welchem $R^{3'}$ für eine niedere Alkylgruppe steht, oder

b) zur Herstellung der Säuren I ohne einen weiteren Reaktionspartner

in Gegenwart wirksamer Mengen eines Komplexes aus einem Palladiumhalogenid und einem tertiären Phosphin (IV) carbonyliert.

**Claim**

A process for the preparation of a 4-substituted but-3-ene-1-carboxylic acid or its esters of the general formula I

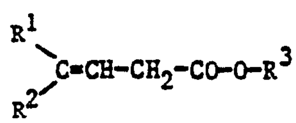

(I)

where $R^1$ is an organic radical, $R^2$ is hydrogen or an organic radical, and $R^1$ and $R^2$ may furthermore be bonded to one another to form a 5-membered to 20-membered ring and should be inert under the reaction conditions specified below, and $R^3$ is hydrogen or lower alkyl, by carbonylation of a derivative of allyl alcohol with the aid of a palladium halide, wherein an allyl alcohol II

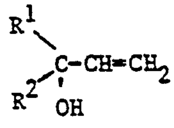

(II)

is carbonylated with carbon monoxide at 50-150 °C and under 200-700 bar,

a) together with an alcohol III

$R^{3'}$—OH (III)

where $R^{3'}$ is lower alkyl, if a predominant amount of an ester I is to be prepared, or

b) without a further reactant where an acid I is to be prepared,

in the presence of an effective amount of a complex of a palladium halide and a tertiary phosphine (IV).

**Revendication**

Procédé de préparation d'acides but-3-ène-1-carboxyliques substitués en position 4 et(ou) d'esters de ceux-ci, de la formule générale I

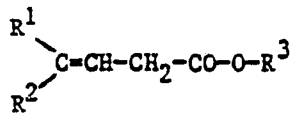

(I)

dans laquelle $R^1$ désigne un groupe organique et $R^2$ un atome d'hydrogène ou un groupe organique, ces groupes devant être inertes dans les conditions opératoires décrites ci-après, $R^1$ et $R^2$ pouvant en outre faire partie d'un noyau cyclique avec cinq à vingt chaînons, et $R^3$ désigne un atome d'hydrogène ou un radical alkyle inférieur, par carbonylation de dérivés de l'alcool allylique à l'aide d'un halogénure du palladium, caractérisé en ce que l'on soumet un alcool allylique II

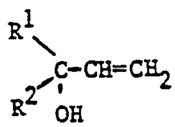

(II)

à une carbonylation par l'oxyde de carbone, entre 50 et 150 °C et sous une pression comprise entre 200 et 700 bars, en présence de quantités efficaces d'un complexe d'un halogénure de palladium et d'une phosphine tertiaire (IV)

a) soit avec un alcool III

$$R^{3'}—OH \qquad (III)$$

pour lequel $R^{3'}$ désigne un radical alkyle inférieur, pour l'obtention de proportions prépondérantes d'esters I ;

b) soit sans autre partenaire de la réaction pour l'obtention des acides I.